# EUROPEAN PATENT SPECIFICATION

(11) **EP 1 996 569 B1**
(45) Date of publication and mention of the grant of the patent: **28.07.2010**
(21) Application number: 07710801.7
(22) Date of filing: 15.03.2007
(51) Int. Cl.: C07D 307/58, A23L 2/56, A23L 1/226, A61Q 11/00, A61K 8/49, C11B 9/00, C11D 3/50

(54) **FLAVORANT AND FRAGRANCE COMPOUNDS**
GESCHMACKS- UND DUFTSTOFFE
COMPOSÉS POUR ARÔME ET PARFUM

(30) Priority: 22.03.2006 US 784813 P
(43) Date of publication of application: 03.12.2008
(73) Proprietor: Givaudan SA, 1214 Vernier-Genève (CH)
(72) Inventor: FURRER, Stefan, Michael, Cincinnati, Ohio 45215 (US); SCHIEBERLE, Peter, 85406 Zolling (DE); BURDACK-FREITAG, Andrea, 83607 Holzkirchen (DE)
(74) Representative: McStea, John Anthony
(86) International application number: PCT/CH2007/000142
(87) International publication number: WO 2007/107023

(56) References cited:
- JP-A- 2003 013 087
- JP-A- 2005 160 402
- US-A- 3 251 366
- YAJIMA I. ET AL: "Volatile Flavor Components of Dried Bonito (Katsuobushi)" AGRICULTURAL AND BIOLOGICAL CHEMISTRY, vol. 47, no. 8, 1983, pages 1755-1760, XP002442053
- SHIMODA M. ET AL: "Comparison of Volatile Compounds among Different Grades of Green Tea and Their Relations to Odor Attributes" JOURNAL OF AGRICULTURAL AND FOOD CHEMISTRY, vol. 43, no. 6, 1995, pages 1621-1625, XP002442054 cited in the application
- CHIACCHIO U ET AL: "A General Synthetic Approach to 5-Alkyl-2(5H)furanones Via 1,3-Dipolar Cycloaddition" TETRAHEDRON, ELSEVIER SCIENCE PUBLISHERS, AMSTERDAM, NL, vol. 54, no. 21, 21 May 1998 (1998-05-21), pages 5695-5708, XP004118419 ISSN: 0040-4020

## Description

This invention relates to flavorants and fragrances and compounds for use therein, especially to a method of providing a flavour or a fragrance to a composition, comprising the addition thereto of a compound of the formula I as defined below.

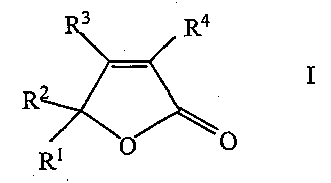

3,5,5-Trimethyl-2(5H)-faranone is known in green tea (see, for example, Shimoda et al, J.Agr.Food.Chem.(1995) 43(6), 1621-5).

The compounds of Formula (I) are selected from those in which the groups R¹, R², R³ and R⁴ in individual compounds are selected as follows:

| R¹ | R² | R³ | R⁴ |
|---|---|---|---|
| Methyl | heptyl | hydrogen | methyl |
| Methyl | Octyl | hydrogen | methyl |
| Methyl | Nonyl | hydrogen | methyl |
| Methyl | Decyl | hydrogen | methyl |
| Methyl | isopentyl | hydrogen | methyl |
| Ethyl | Ethyl | hydrogen | methyl |
| Ethyl | Propyl | hydrogen | methyl |
| Ethyl | Pentyl | hydrogen | methyl |
| Ethyl | Hexyl | hydrogen | methyl |
| Ethyl | Heptyl | hydrogen | methyl |
| Ethyl | Octyl | hydrogen | methyl |
| Ethyl | Nonyl | hydrogen | methyl |
| Ethyl | Decyl | hydrogen | methyl |
| 3-membered ring with R2 | | hydrogen | methyl |
| 4-membered ring with R2 | | hydrogen | methyl |
| 5-membered ring with R2 | | hydrogen | methyl |
| 7- membered ring with R2 | | hydrogen | methyl |
| Methyl | Methyl | hydrogen | Pentyl |
| Methyl | Methyl | hydrogen | Hexyl |
| Methyl | Methyl | hydrogen | Heptyl |
| Methyl | Methyl | hydrogen | Octyl |
| Methyl | Methyl | hydrogen | Nonyl |
| Methyl | Methyl | hydrogen | Decyl |
| Methyl | Hexyl | hydrogen | Methyl |
| Methyl | n-Butyl | hydrogen | Methyl |

the compounds may be obtained from natural sources or synthesised by known methods, for example, by that described by Iwahama et al in Chem. Comm., 2000, 613-614 and Yates in Can. J. Chem., 1987, 65 (8), 1695.

The compounds of Formula I are useful for the provision of flavours in compositions that are to be taken orally. Thus, in certain embodiments, a composition for oral reception or ingestion is provided, the composition comprising a flavorant comprising a compound of Formula I as hereinabove described. The composition to which the compound of Formula I is added may be any such composition. For example, it may be a foodstuff, baked goods, confectionery, including chewing gum and hard candy, a beverage, a tobacco product, a dental preparation, such as a toothpaste, a tooth gel or a mouthwash, or a solid, liquid or sprayable medicinal preparation. Therefore, also provided is a flavoured product that is orally receivable or ingestible, in which the flavoured product comprises a flavorant that is a compound according to Formula I above.

The compounds of Formula I are also useful as fragrances. They may be used in fine fragrances, such as perfumes, or as functional fragrances in any of the wide range of products that contain fragrance, for example, detergents, soaps and other personal products, such as creams and lotions, body care products, cosmetic products, household cleaning agents and preparations, polishes, consumer healthcare products, air fresheners and the like. Therefore, further provided is a fragranced product, in which the fragranced product comprises a fragrance that is a compound according to Formula I above.

The phrases "a compound of Formula I" and "a compound according to Formula I" refer to embodiments in which one such compound, or a mixture or combination of more than one such compound, is used.

The compounds of Formula I may be added to products in the conventional manner using standard equipment and in generally art-recognised proportions. These will naturally vary, depending on the use and extent of flavour or fragrance desired. It is also possible to exceed the generally-recognised proportions, should a particular effect should be desired. In addition, such products may also incorporate the particular raw materials known to the art for use in that particular product, again in the usual proportions.

The method, compounds, flavoured compositions, and fragranced compositions are further described with reference to the following non-limiting, illustrative examples.
5-butyl-3,5-dimethylfuran-2(5H)-one
5-hexyl-3,5-dimethylfuran-2(5H)-one
5-isopentyl-3,5-dimethylfuran-2(5H)-one
3,5-dimethyl-5-octylfuran-2(5H)-one
5-decyl-3,5-dimethylfuran-2(5H)-one
3-hexyl-5,5-dimethylfuran-2(5H)-one
5,5-diethyl-3-methylfuran-2(5H)-one
3-methyl-l-oxaspiro[4.5]dec-3-en-2-one
3-methyl-1-Oxaspiro[4.4]non-3-en-2-one

Certain exemplary compounds and their characteristics are shown bellow, followed by the method of preparation:

### Example

| | | |
|---|---|---|
| Taste description (at 10ppm in water): nutty, brow, condensed milk, nutmeat, slight fatty mouthfeel, and added slight astringent aftertaste. | 1* | 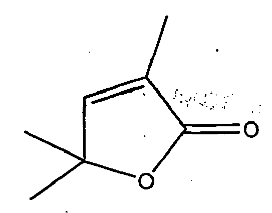 |
| Taste description (at 1ppm in water): Mushroom, musty, oily mouthfeel, greasy, and added astringent aftertaste. | 2 | 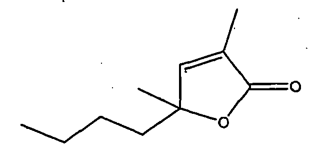 |

| | | |
|---|---|---|
| *not part of the invention | | |

Methods for the general preparation of unsaturated lactones from α-hydroxy-γ-lactones are described in T. Iwahama, S. Sakaguchi, Y. Ishii, Chem. Comm., 2000, 613-614 and P. Yates, Can. J. Chem., 1987, 65 (8), 1695.

### Example 1: 3,5,5-tiimethylfuran-2(5H)-one (not part of the invention)

### A) Preparation of dihydro-3-hydroxy-3,5,5-trimethylfuran-2(3H)-one

In a 500mL flask, fitted with magnetic stirrer, reflux condenser, 3.26g of N-hydroxyphtalimide, 120g of isopropanol, 0.05g of Cobalt (II) acetate (4H₂O) and 0.71g of cobalt (III)acetylacetonate and 33mL of acetonitrile were added. The flask was evacuated and placed under an oxygen atmosphere (balloon). 20g of methyl methacrylate were added at room temperature and the greenish solution was heated to 70°C and stirred at 70°C for 6h and for 16h (over night) at RT (under nitrogen), while it became brownish.

The mixture was concentrated and purified by column chromatography and crystallization from MTBE/Hexane, yielding 5.63g white crystals (19.5% yield).
¹H NMR (300 MHz; CDCl₃) δ: 2.75 (s, 1H) 2.32 (d, 1H), 2.10 (d, 1H), 1.53 (d, 6H), 1.46 (s, 3H)
¹³C NMR (300 MHz; CDCl₃) δ: 178.49, 82.21, 48.45, 29.13, 28.98, 26.30 mp:66-68°C

### B) Preparation of 3,5,5-trimethylfuran-2(5H)-one

In a 100mL flask, fitted with magnetic stirrer, reflux condenser, 5.00g of dihydro-3-hydroxy-3,5,5-trimethylfuran-2(3H)-one and 29.2g of hydrobromic acid (48% in water) were added and heated at reflux (135°C oilbath) for 4h. The mixture was poured on ice and extracted with MTBE. The organic layer was washed with brine, dried over magnesium sulfate and concentrated. The crude product was crystallized from MTBE and washed with hexane, yielding 1.75g white crystals (40% yield).
¹H NMR (300 MHz; CDCl₃) δ: 7.00 (dd, 1H), 1.89 (d, 3H), 1.44 (s, 6H)
¹³C NMR (300 MHz; CDCl₃) δ: 173.63, 153.92, 128.18, 84.06, 25.63, 10.41
MS: 126 (M+), 111, 83, 67, 55, 43, 39
IR: 3462.2, 3087.9, 2983.0, 2932.1, 1744.3, 1665.7
mp: 54-55°C

### Example 2: 5-butyl-3,5-dimethylfuran-2(5H)-one

5-butyl-3,5-dimethylfuran-2(5H)-one was prepared according to the general procedure in Example 1, using 2-hexanol and methyl methacrylate.
¹H NMR (300 MHz; CDCl₃) δ: 6.9 (dd, 1H), 1.90 (d, 3H), 1.8-1.6 (m, 2H), 1.41 (s, 3H), 1.3-1.2 (m, 4H), 0.9 (t, 3H)
¹³C NMR (300 MHz; CDCl₃) δ: 173.83, 153.06, 128.75, 86.47, 38.36, 24.14, 22.73, 13.83, 10.47
MS: 168 (M+), 153,140,125,111,97, 83,69, 55, 43

### Use-Example : Hazelnut

10ppm of 3,5,5-trimethylfuran-2(5H)-one (Example 1) was added to a beverage base (10g sugar, 90g water) containing 0.65% by weight of hazelnut fluid extract (a hydroalcoholic extract (ethanol and propylene glycol) of hazelnut, *corylus Americana*, ex Chart). The resulting composition showed a fuller, toasted, slight roasted, brown, slightly creamy, definitely nutty, slight macadamia nut aroma, while the hazelnut extract alone was slightly sweet, woody, astringent and rather plain at that level.

## Claims

1. A method of providing a flavour or a fragrance to a composition, comprising adding to said composition a compound of the Formula I 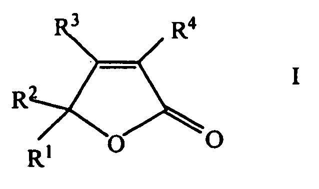 wherein R¹, R², R³ and R⁴ for individual compounds are selected as follows:
| R¹ | R² | R³ | R⁴ |
|---|---|---|---|
| Methyl | heptyl | H | methyl |
| Methyl | Octyl | H | methyl |
| Methyl | Nonyl | H | methyl |
| Methyl | Decyl | H | methyl |
| Methyl | isopentyl | H | methyl |
| Ethyl | Ethyl | H | methyl |
| Ethyl | Propyl | H | methyl |
| Ethyl | Pentyl | H | methyl |
| Ethyl | Hexyl | H | methyl |
| Ethyl | Heptyl | H | methyl |
| Ethyl | Octyl | H | methyl |
| Ethyl | Nonyl | H | methyl |
| Ethyl | Decyl | H | methyl |
| 3-membered ring with R2 | | H | methyl |
| 4-membered ring with R2 | | H | methyl |
| 5-membered ring with R2 | | H | methyl |
| 7- membered ring with R2 | | H | methyl |
| Methyl | Methyl | H | Pentyl |
| Methyl | Methyl | H | Hexyl |
| Methyl | Methyl | H | Heptyl |
| Methyl | Methyl | H | Octyl |
| Methyl | Methyl | H | Nonyl |
| Methyl | Methyl | H | Decyl |
| Methyl | Hexyl | H | Methyl |
| Methyl | n-Butyl | H | Methyl |

2. A compound of Formula I 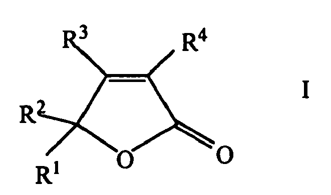 wherein R¹, R², R³ and R⁴ for individual compounds are selected as follows:
| R¹ | R² | R³ | R⁴ |
|---|---|---|---|
| Methyl | heptyl | H | methyl |
| Methyl | Octyl | H | methyl |
| Methyl | Nonyl | H | methyl |
| Methyl | Decyl | H | methyl |
| Methyl | isopentyl | H | methyl |
| Ethyl | Ethyl | H | methyl |
| Ethyl | Propyl | H | methyl |
| Ethyl | Pentyl | H | methyl |
| Ethyl | Hexyl | H | methyl |
| Ethyl | Heptyl | H | methyl |
| Ethyl | Octyl | H | methyl |
| Ethyl | Nonyl | H | methyl |
| Ethyl | Decyl | H | methyl |
| 3-membered ring with R2 | | H | methyl |
| 4-membered ring with R2 | | H | methyl |
| 5-membered ring with R2 | | H | methyl |
| 7- membered ring with R2 | | H | methyl |
| Methyl | Methyl | H | Pentyl |
| Methyl | Methyl | H | Hexyl |
| Methyl | Methyl | H | Heptyl |
| Methyl | Methyl | H | Octyl |
| Methyl | Methyl | H | Nonyl |
| Methyl | Methyl | H | Decyl |
| Methyl | Hexyl | H | Methyl |
| Methyl | n-Butyl | H | Methyl |

3. A flavored product that is orally receivable or ingestible, comprising a flavorant that is a compound of Formula I: 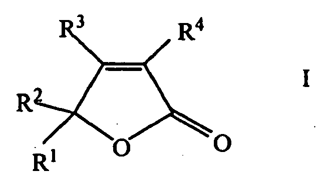 according to claim 1.

4. A fragranced product, comprising a fragrance that is a compound of Formula I: 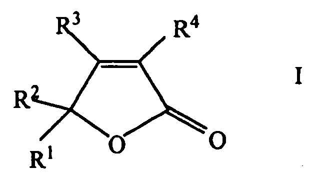 according to claim 1.

## Patentansprüche

1. Verfahren zum Versehen einer Zusammensetzung mit einem Geschmack oder Duft, bei dem man die Zusammensetzung mit einer Verbindung der Formel I 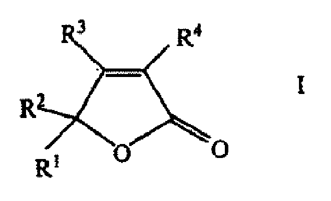 worin R¹, R², R³ und R⁴ für einzelne Verbindungen wie folgt gewählt sind:
| R¹ | R² | R³ | R⁴ |
|---|---|---|---|
| Methyl | Heptyl | H | Methyl |
| Methyl | Octyl | H | Methyl |
| Methyl | Nonyl | H | Methyl |
| Methyl | Decyl | H | Methyl |
| Methyl | Isopentyl | H | Methyl |
| Ethyl | Ethyl | H | Methyl |
| Ethyl | Propyl | H | Methyl |
| Ethyl | Pentyl | H | Methyl |
| Ethyl | Hexyl | H | Methyl |
| Ethyl | Heptyl | H | Methyl |
| Ethyl | Octyl | H | Methyl |
| Ethyl | Nonyl | H | Methyl |
| Ethyl | Decyl | H | Methyl |
| 3-gliedriger Ring mit R2 | | H | Methyl |
| 4-gliedriger Ring mit R2 | | H | Methyl |
| 5-gliedriger Ring mit R2 | | H | Methyl |
| 7-gliedriger Ring mit R2 | | H | Methyl |
| Methyl | Methyl | H | Pentyl |
| Methyl | Methyl | H | Hexyl |
| Methyl | Methyl | H | Heptyl |
| Methyl | Methyl | H | Octyl |
| Methyl | Methyl | H | Nonyl |
| Methyl | Methyl | H | Decyl |
| Methyl | Hexyl | H | Methyl |
| Methyl | n-Butyl | H | Methyl |
versetzt.

2. Verbindung der Formel I 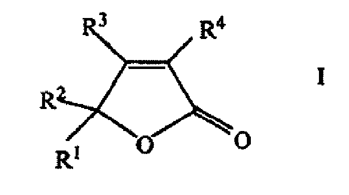 worin R¹, R², R³ und R⁴ für einzelne Verbindungen wie folgt gewählt sind:
| R¹ | R² | R³ | R⁴ |
|---|---|---|---|
| Methyl | Heptyl | H | Methyl |
| Methyl | Octyl | H | Methyl |
| Methyl | Nonyl | H | Methyl |
| Methyl | Decyl | H | Methyl |
| Methyl | Isopentyl | H | Methyl |
| Ethyl | Ethyl | H | Methyl |
| Ethyl | Propyl | H | Methyl |
| Ethyl | Pentyl | H | Methyl |
| Ethyl | Hexyl | H | Methyl |
| Ethyl | Heptyl | H | Methyl |
| Ethyl | Octyl | H | Methyl |
| Ethyl | Nonyl | H | Methyl |
| Ethyl | Decyl | H | Methyl |
| 3-gliedriger Ring mit R2 | | H | Methyl |
| 4-gliedriger Ring mit R2 | | H | Methyl |
| 5-gliedriger Ring mit R2 | | H | Methyl |
| 7-gliedriger Ring mit R2 | | H | Methyl |
| Methyl | Methyl | H | Pentyl |
| Methyl | Methyl | H | Hexyl |
| Methyl | Methyl | H | Heptyl |
| Methyl | Methyl | H | Octyl |
| Methyl | Methyl | H | Nonyl |
| Methyl | Methyl | H | Decyl |
| Methyl | Hexyl | H | Methyl |
| Methyl | n-Butyl | H | Methyl |

3. Oral auf- bzw. einnehmbares geschmacksstoffhaltiges Produkt, enthaltend einen Geschmacksstoff, bei dem es sich um eine Verbindung der Formel I: 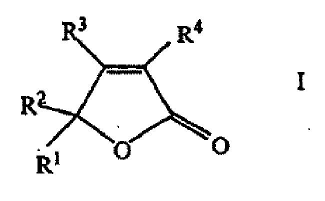 nach Anspruch 1 handelt.

4. Duftstoffhaltiges Produkt, enthaltend einen Duftstoff, bei dem es sich um eine Verbindung der Formel I: 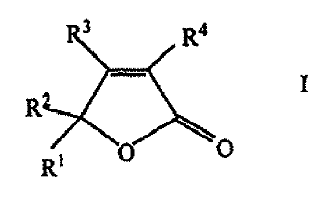 nach Anspruch 1 handelt.

## Revendications

1. Méthode pour donner un arôme ou un parfum à une composition, comprenant l'addition à ladite composition d'un composé de formule I 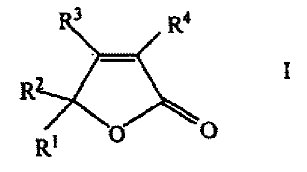 dans laquelle R¹, R² R³ et R⁴ pour les composés individuels sont choisis comme suait :
| R¹ | R² | R³ | R⁴ |
|---|---|---|---|
| Méthyle | heptyle | H | méthyle |
| Méthyle | Octyle | H | méthyle |
| Méthyle | Nonyle | H | méthyle |
| Méthyle | Décyle | H | méthyle |
| Méthyle | isopentyle | H | méthyle |
| Ethyle | Ethyle | H | méthyle |
| Ethyle | Propyle | H | méthyle |
| Ethyle | Pentyle | H | méthyle |
| Ethyle | Hexyle | H | méthyle |
| Ethyle | Heptyle | H | méthyle |
| Ethyle | Octyle | H | méthyle |
| Ethyle | Nonyle | H | méthyle |
| Ethyle | Décyle | H | méthyle |
| Cycle à 3 chaînons avec R2 | | H | méthyle |
| Cycle à 4 chaînons avec R2 | | H | méthyle |
| Cycle à 5 chaînons avec R2 | | H | méthyle |
| Cycle à 7 chaînons avec R2 | | H | méthyle |
| Méthyle | Méthyle | H | Pentyle |
| Méthyle | Méthyle | H | Hexyle |
| Méthyle | Méthyle | H | Heptyle |
| Méthyle | Méthyle | H | Octyle |
| Méthyle | Méthyle | H | Nonyle |
| Méthyle | Méthyle | H | Décyle |
| Méthyle | Hexyle | H | Méthyle |
| Méthyle | n-Butyle | H | Méthyle |

2. Composé de formule I 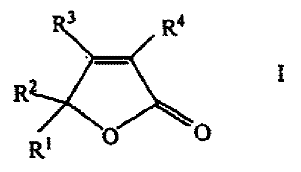 dans laquelle R¹, R², R³ et R⁴ pour les composés individuels sont choisis comme suait :
| R¹ | R² | R³ | R⁴ |
|---|---|---|---|
| Méthyle | heptyle | H | méthyle |
| Méthyle | Octyle | H | méthyle |
| Méthyle | Nonyle | H | méthyle |
| Méthyle | Décyle | H | méthyle |
| Méthyle | isopentyle | H | méthyle |
| Ethyle | Ethyle | H | méthyle |
| Ethyle | Propyle | H | méthyle |
| Ethyle | Pentyle | H | méthyle |
| Ethyle | Hexyle | H | méthyle |
| Ethyle | Heptyle | H | méthyle |
| Ethyle | Octyle | H | méthyle |
| Ethyle | Nonyle | H | méthyle |
| Ethyle | Décyle | H | méthyle |
| Cycle à 3 chaînons avec R2 | | H | méthyle |
| Cycle à 4 chaînons avec R2 | | H | méthyle |
| Cycle à 5 chaînons avec R2 | | H | méthyle |
| Cycle à 7 chaînons avec R2 | | H | méthyle |
| Méthyle | Méthyle | H | Pentyle |
| Méthyle | Méthyle | H | Hexyle |
| Méthyle | Méthyle | H | Heptyle |
| Méthyle | Méthyle | H | Octyle |
| Méthyle | Méthyle | H | Nonyle |
| Méthyle | Méthyle | H | Décyle |
| Méthyle | Hexyle | H | Méthyle |
| Méthyle | n-Butyle | H | Méthyle |

3. Produit aromatisé qui est recevable ou ingérable par voie orale, comprenant un aromatisant qui est un composé de formule I : 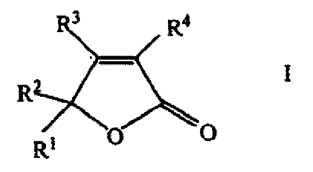 selon la revendication 1.

4. Produit parfumé, comprenant un parfum qui est un composé de formule I : 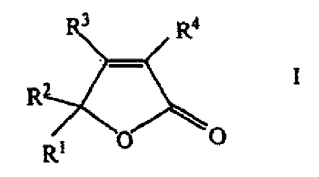 selon la revendication 1.
